(19) Europäisches Patentamt
European Patent Office
Office européen des brevets

(11) EP 1 266 008 B1

(12) EUROPEAN PATENT SPECIFICATION

(45) Date of publication and mention of the grant of the patent:
**30.08.2006 Bulletin 2006/35**

(21) Application number: **01936104.7**

(22) Date of filing: **20.03.2001**

(51) Int Cl.:
*C12N 15/12* (2006.01) *C07K 14/47* (2006.01)
*C12N 15/62* (2006.01) *C12Q 1/68* (2006.01)
*C12N 5/10* (2006.01) *C07K 16/18* (2006.01)
*G01N 33/68* (2006.01)

(86) International application number:
**PCT/EP2001/003149**

(87) International publication number:
**WO 2001/070771 (27.09.2001 Gazette 2001/39)**

# (54) ACUTE NEURONAL INDUCED CALCIUM BINDING PROTEIN TYPE 1 LIGAND

AKUTER NEURONAL-INDUZIERTER LIGANDEN DES CALCIUM-BINDENDEN PROTEIN-1

LIGAND DE PROTEINE FIXANT LE CALCIUM A INDUCTION NEURONALE AIGUE DE TYPE 1

(84) Designated Contracting States:
**AT BE CH CY DE DK ES FI FR GB GR IE IT LI LU MC NL PT SE TR**

(30) Priority: **21.03.2000 EP 00106110**

(43) Date of publication of application:
**18.12.2002 Bulletin 2002/51**

(73) Proprietor: **Merck Patent GmbH**
**64293 Darmstadt (DE)**

(72) Inventors:
- **DEN DAAS, Izaak**
  **64407 Fränkisch-Crumbach (DE)**
- **DÜCKER, Klaus**
  **64291 Darmstadt (DE)**
- **HOCK, Björn**
  **63477 Maintal (DE)**

(56) References cited:
- **DATABASE EMBL [Online] 10 April 1998 (1998-04-10) NAGASE,T. ET AL.0: "KIAA0524 PROTEIN (FRAGMENT)." retrieved from EBI Database accession no. AB011096 XP000884356 cited in the application -& DATABASE TREMBL [Online] 1 August 1998 (1998-08-01) retrieved from EBI Database accession no. O60277 XP002187866 cited in the application**
- **MINK MATYAS ET AL: "A novel human gene (SARM) at chromosome 17q11 encodes a protein with a SAM motif and structural similarity to Armadillo/beta-catenin that is conserved in mouse, Drosophila, and Caenorhabditis elegans." GENOMICS, vol. 74, no. 2, 1 June 2001 (2001-06-01), pages 234-244, XP002187847 ISSN: 0888-7543**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

SEQUENCE LISTING

<110> Merck Patent GmbH

<120> ANIC-BP1-ligand

<130> ANIC-BP-1-ligand

<140>
<141>

<160> 8

<170> Patent In Ver. 2.1

<210> 1
<211> 2700
<212> DNA
<213> Homo sapiens

<220>
<221> CDS
<222> (363)..(2432)

<220>
<223> Description of ANIC-BP-1 protein ligand

<400> 1

```
agccatccct cgcctgctcg ctctctcctt tcgcccactc cctgcatctg ggcctgcatc 60

acctttgcca accgctcccc cgatcctgcc gacactcctc ccccaaactt ctgaccggca 120

cccttgcctg gtacccttct ctccattcct ccccctccat cttctttccc cgacccctct 180

cgggtccctc ttttcccaaa acccgggtct ctccgcgtgg ccccgcctcc aggccgggga 240

tgtcccccgc ggccccgcgc ccatggtcct gacgctgctt ctctccgcct acaagctgtg 300

tcgcttcttc gccatgtcgg gcccacggcg gggcgccgag cggctggcgg tgcctgggcc 360

ag atg ggg gcg gtg gca cgg gcc cat ggt ggg ctg cgg gtg gcc cgg    407
   Met Gly Ala Val Ala Arg Ala His Gly Gly Leu Arg Val Ala Arg
   1               5                   10                  15

gcc cgc gaa agt gtc gcc ggg ggc agg cac cga ggt gca gga cgc cct   455
Ala Arg Glu Ser Val Ala Gly Gly Arg His Arg Gly Ala Gly Arg Pro
                20                  25                  30

gga gcg cgc gct gcc gga gct gca gca ggc ctt gtc cgc gct gaa gca   503
Gly Ala Arg Ala Ala Gly Ala Ala Ala Gly Leu Val Arg Ala Glu Ala
            35                  40                  45

ggc ggg cgg cgc gcg ggc cgt ggg cgc cgg cct ggc cga ggt ctt cca   551
Gly Gly Arg Arg Ala Gly Arg Gly Arg Arg Pro Gly Arg Gly Leu Pro
        50                  55                  60

act ggt gga gga ggc ctg gct gct gcg gcc gtg ggc cgc gag gta gcc   599
Thr Gly Gly Gly Gly Leu Ala Ala Ala Ala Val Gly Arg Glu Val Ala
    65                  70                  75

cag ggt ctg tgc gac gcc atc cgc ctc gat ggc ggc ctc gac ctg ctg   647
```

```
Gln Gly Leu Cys Asp Ala Ile Arg Leu Asp Gly Gly Leu Asp Leu Leu
 80                 85                  90                  95

ttg cgg ctg ctg cag gcg ccg gag ttg gag acg cgt gtg cag gcc gcg    695
Leu Arg Leu Leu Gln Ala Pro Glu Leu Glu Thr Arg Val Gln Ala Ala
                100                 105                 110

cgc ctg ctg gag cag atc ctg gtg gct gag aac cga gac cgc gtg gcg    743
Arg Leu Leu Glu Gln Ile Leu Val Ala Glu Asn Arg Asp Arg Val Ala
            115                 120                 125

cgc att ggg ctg ggc gtg atc ctg aac ctg gcg aag gaa cgc gaa ccc    791
Arg Ile Gly Leu Gly Val Ile Leu Asn Leu Ala Lys Glu Arg Glu Pro
        130                 135                 140

gta gag ctg gcg cgg agt ggg tca ggc atc ttg gag cac atg ttc aag    839
Val Glu Leu Ala Arg Ser Gly Ser Gly Ile Leu Glu His Met Phe Lys
    145                 150                 155

cat tcg gag gag aca tgc cag agg ctg gtg gcg gcc ggc ggc ctg gac    887
His Ser Glu Glu Thr Cys Gln Arg Leu Val Ala Ala Gly Gly Leu Asp
160                 165                 170                 175

gcg gtg ctg tat tgg tgc cgc cgc acg gac ccc gcg ctg ctg cgc cac    935
Ala Val Leu Tyr Trp Cys Arg Arg Thr Asp Pro Ala Leu Leu Arg His
                180                 185                 190

tgc gcg ctg gcg ctg ggc aac tgc gcg ctg cac ggg ggc cag gcg gtg    983
Cys Ala Leu Ala Leu Gly Asn Cys Ala Leu His Gly Gly Gln Ala Val
            195                 200                 205

cag cga cgc atg gta gag aag cgc gca gcc gag tgg ctc ttc ccg ctc    1031
Gln Arg Arg Met Val Glu Lys Arg Ala Ala Glu Trp Leu Phe Pro Leu
        210                 215                 220

gcc ttc tcc aag gag gac gag ctg ctt tcg ctg cac gcc tgc ctc gca    1079
Ala Phe Ser Lys Glu Asp Glu Leu Leu Ser Leu His Ala Cys Leu Ala
    225                 230                 235

gta gcg gtg ttg gcg act aac aag gag gtg gag cgc gag gtg gag cgc    1127
Val Ala Val Leu Ala Thr Asn Lys Glu Val Glu Arg Glu Val Glu Arg
240                 245                 250                 255

tcg ggc acg ctg gcg ctc gtg gag ccg ctt gtg gcc tcg ctg gac cct    1175
Ser Gly Thr Leu Ala Leu Val Glu Pro Leu Val Ala Ser Leu Asp Pro
                260                 265                 270

ggc cgc ttc gcc cgc tgt ctg gtg gac gcc agc gac aca agc cag ggc    1223
Gly Arg Phe Ala Arg Cys Leu Val Asp Ala Ser Asp Thr Ser Gln Gly
            275                 280                 285

cgc ggg ccc gac gac ctg cag cgc ctc gtg ccg ttg ctc gac tct aac    1271
Arg Gly Pro Asp Asp Leu Gln Arg Leu Val Pro Leu Leu Asp Ser Asn
        290                 295                 300

cgc ttg gag gcg cag tgc atc ggg gct ttc tac ctc tgc gcc gag gct    1319
Arg Leu Glu Ala Gln Cys Ile Gly Ala Phe Tyr Leu Cys Ala Glu Ala
    305                 310                 315

gcc atc aag agc ctg caa ggc aag acc aag gtg ttc agc gac atc ggc    1367
Ala Ile Lys Ser Leu Gln Gly Lys Thr Lys Val Phe Ser Asp Ile Gly
```

```
320                 325                 330                 335

gcc atc cag agc ctg aaa cgc ctg gtt tcc tac tct acc aat ggc act     1415
Ala Ile Gln Ser Leu Lys Arg Leu Val Ser Tyr Ser Thr Asn Gly Thr
                340                 345                 350

aag tcg gcg ctg gcc aag cgc gcg ctg cgc ctg ctg ggc gag gag gtg     1463
Lys Ser Ala Leu Ala Lys Arg Ala Leu Arg Leu Leu Gly Glu Glu Val
            355                 360                 365

cca cgg ccc atc ctg ccc tcc gtg ccc agc tgg aag gag gcc gag gtt     1511
Pro Arg Pro Ile Leu Pro Ser Val Pro Ser Trp Lys Glu Ala Glu Val
        370                 375                 380

cag acg tgg ctg cag cag atc ggt ttc tcc aag tac tgc gag agc ttc     1559
Gln Thr Trp Leu Gln Gln Ile Gly Phe Ser Lys Tyr Cys Glu Ser Phe
    385                 390                 395

cgg gag cag cag gtg gat ggc gac ctg ctt ctg cgg ctc acg gag gag     1607
Arg Glu Gln Gln Val Asp Gly Asp Leu Leu Leu Arg Leu Thr Glu Glu
400                 405                 410                 415

gaa ctc cag acc gac ctg ggc atg aaa tcg ggc atc acc cgc aag agg     1655
Glu Leu Gln Thr Asp Leu Gly Met Lys Ser Gly Ile Thr Arg Lys Arg
                420                 425                 430

ttc ttt agg gag ctc acg gag ctc aag acc ttc gcc aac tat tct acg     1703
Phe Phe Arg Glu Leu Thr Glu Leu Lys Thr Phe Ala Asn Tyr Ser Thr
            435                 440                 445

tgc gac cgc agc aac ctg gcg gac tgg ctg ggc agc ctg gac ccg cgc     1751
Cys Asp Arg Ser Asn Leu Ala Asp Trp Leu Gly Ser Leu Asp Pro Arg
        450                 455                 460

ttc cgc cag tac acc tac ggc ctg gtc agc tgc ggc ctg gac cgc tcc     1799
Phe Arg Gln Tyr Thr Tyr Gly Leu Val Ser Cys Gly Leu Asp Arg Ser
    465                 470                 475

ctg ctg cac cgc gtg tct gag cag cag ctg ctg gaa gac tgc ggc atc     1847
Leu Leu His Arg Val Ser Glu Gln Gln Leu Leu Glu Asp Cys Gly Ile
480                 485                 490                 495

cac ctg ggc gtg cac cgc gcc cgc atc ctc acg gcg gcc aga gaa atg     1895
His Leu Gly Val His Arg Ala Arg Ile Leu Thr Ala Ala Arg Glu Met
                500                 505                 510

cta cac tcc ccg ctg ccc tgt act ggt ggc aaa ccc agt ggg gac act     1943
Leu His Ser Pro Leu Pro Cys Thr Gly Gly Lys Pro Ser Gly Asp Thr
            515                 520                 525

cca gat gtc ttc atc agc tac cgc cgg aac tca ggt tcc cag ctg gcc     1991
Pro Asp Val Phe Ile Ser Tyr Arg Arg Asn Ser Gly Ser Gln Leu Ala
        530                 535                 540

agt ctc ctg aag gtg cac ctg cag ctg cat ggc ttc agt gtc ttc att     2039
Ser Leu Leu Lys Val His Leu Gln Leu His Gly Phe Ser Val Phe Ile
    545                 550                 555

gat gtg gag aag ctg gaa gca ggc aag ttc gag gac aaa ctc atc cag     2087
Asp Val Glu Lys Leu Glu Ala Gly Lys Phe Glu Asp Lys Leu Ile Gln
560                 565                 570                 575
```

```
agt gtc atg ggt gcc cgc aac ttt gtg ttg gtg cta tca cct gga gca     2135
Ser Val Met Gly Ala Arg Asn Phe Val Leu Val Leu Ser Pro Gly Ala
                580                 585                 590

ctg gac aag tgc atg caa gac cat gac tgc aag gat tgg gtg cat aag     2183
Leu Asp Lys Cys Met Gln Asp His Asp Cys Lys Asp Trp Val His Lys
            595                 600                 605

gag att gtg act gct tta agc tgc ggc aag aac att gtg ccc atc att     2231
Glu Ile Val Thr Ala Leu Ser Cys Gly Lys Asn Ile Val Pro Ile Ile
        610                 615                 620

gat ggc ttc gag tgg cct gag ccc cag gtc ctg cct gag gac atg cag     2279
Asp Gly Phe Glu Trp Pro Glu Pro Gln Val Leu Pro Glu Asp Met Gln
    625                 630                 635

gct gtg ctt act ttc aac ggt atc aag tgg tcc cac gaa tac cag gag     2327
Ala Val Leu Thr Phe Asn Gly Ile Lys Trp Ser His Glu Tyr Gln Glu
640                 645                 650                 655

gcc acc att gag aag atc atc cgc ttc ctg cag ggc cgc tcc tcc cgg     2375
Ala Thr Ile Glu Lys Ile Ile Arg Phe Leu Gln Gly Arg Ser Ser Arg
                660                 665                 670

gac tca tct gca ggc tct gac acc agt ttg gag ggt gct gca ccc atg     2423
Asp Ser Ser Ala Gly Ser Asp Thr Ser Leu Glu Gly Ala Ala Pro Met
            675                 680                 685

ggt cca acc taaccagtcc ccagttcccc agccctgctg tgacttccat             2472
Gly Pro Thr
        690

ttccatcgtc ctttctgaag gaacagctcc tgaaaccagt ctccctgggc tgagacaacc 2532

tgggctcttc ttaggaaatg gctctccctc cccctgtccc ccaccctcat ggcccacctc 2592

caacccactt tcctcagtat ctggagaggg aagggaagtc aggcttgggc acgggaggtt 2652

agaactcccc caggccctgc cattgggttg tctgtctccg tcatgggg               2700
```

<210> 2
<211> 690
<212> PRT
<213> Homo sapiens
<223> Description of ANIC-BP-1 protein ligand

<400> 2

```
Met Gly Ala Val Ala Arg Ala His Gly Gly Leu Arg Val Ala Arg Ala
 1               5                  10                  15

Arg Glu Ser Val Ala Gly Gly Arg His Arg Gly Ala Gly Arg Pro Gly
            20                  25                  30

Ala Arg Ala Ala Gly Ala Ala Ala Gly Leu Val Arg Ala Glu Ala Gly
        35                  40                  45

Gly Arg Arg Ala Gly Arg Gly Arg Arg Pro Gly Arg Gly Leu Pro Thr
    50                  55                  60
```

Gly Gly Gly Gly Leu Ala Ala Ala Ala Val Gly Arg Glu Val Ala Gln
65                  70                  75                  80

Gly Leu Cys Asp Ala Ile Arg Leu Asp Gly Gly Leu Asp Leu Leu Leu
                85                  90                  95

Arg Leu Leu Gln Ala Pro Glu Leu Glu Thr Arg Val Gln Ala Ala Arg
            100                 105                 110

Leu Leu Glu Gln Ile Leu Val Ala Glu Asn Arg Asp Arg Val Ala Arg
        115                 120                 125

Ile Gly Leu Gly Val Ile Leu Asn Leu Ala Lys Glu Arg Glu Pro Val
    130                 135                 140

Glu Leu Ala Arg Ser Gly Ser Gly Ile Leu Glu His Met Phe Lys His
145                 150                 155                 160

Ser Glu Glu Thr Cys Gln Arg Leu Val Ala Ala Gly Gly Leu Asp Ala
                165                 170                 175

Val Leu Tyr Trp Cys Arg Arg Thr Asp Pro Ala Leu Leu Arg His Cys
            180                 185                 190

Ala Leu Ala Leu Gly Asn Cys Ala Leu His Gly Gly Gln Ala Val Gln
        195                 200                 205

Arg Arg Met Val Glu Lys Arg Ala Ala Glu Trp Leu Phe Pro Leu Ala
    210                 215                 220

Phe Ser Lys Glu Asp Glu Leu Leu Ser Leu His Ala Cys Leu Ala Val
225                 230                 235                 240

Ala Val Leu Ala Thr Asn Lys Glu Val Glu Arg Glu Val Glu Arg Ser
                245                 250                 255

Gly Thr Leu Ala Leu Val Glu Pro Leu Val Ala Ser Leu Asp Pro Gly
            260                 265                 270

Arg Phe Ala Arg Cys Leu Val Asp Ala Ser Asp Thr Ser Gln Gly Arg
        275                 280                 285

Gly Pro Asp Asp Leu Gln Arg Leu Val Pro Leu Leu Asp Ser Asn Arg
    290                 295                 300

Leu Glu Ala Gln Cys Ile Gly Ala Phe Tyr Leu Cys Ala Glu Ala Ala
305                 310                 315                 320

Ile Lys Ser Leu Gln Gly Lys Thr Lys Val Phe Ser Asp Ile Gly Ala
                325                 330                 335

Ile Gln Ser Leu Lys Arg Leu Val Ser Tyr Ser Thr Asn Gly Thr Lys
            340                 345                 350

Ser Ala Leu Ala Lys Arg Ala Leu Arg Leu Leu Gly Glu Glu Val Pro
        355                 360                 365

Arg Pro Ile Leu Pro Ser Val Pro Ser Trp Lys Glu Ala Glu Val Gln
    370                 375                 380

Thr Trp Leu Gln Gln Ile Gly Phe Ser Lys Tyr Cys Glu Ser Phe Arg

```
385                 390                 395                 400

Glu Gln Gln Val Asp Gly Asp Leu Leu Leu Arg Leu Thr Glu Glu Glu
                405                 410                 415

Leu Gln Thr Asp Leu Gly Met Lys Ser Gly Ile Thr Arg Lys Arg Phe
            420                 425                 430

Phe Arg Glu Leu Thr Glu Leu Lys Thr Phe Ala Asn Tyr Ser Thr Cys
        435                 440                 445

Asp Arg Ser Asn Leu Ala Asp Trp Leu Gly Ser Leu Asp Pro Arg Phe
    450                 455                 460

Arg Gln Tyr Thr Tyr Gly Leu Val Ser Cys Gly Leu Asp Arg Ser Leu
465                 470                 475                 480

Leu His Arg Val Ser Glu Gln Gln Leu Leu Glu Asp Cys Gly Ile His
                485                 490                 495

Leu Gly Val His Arg Ala Arg Ile Leu Thr Ala Ala Arg Glu Met Leu
            500                 505                 510

His Ser Pro Leu Pro Cys Thr Gly Gly Lys Pro Ser Gly Asp Thr Pro
        515                 520                 525

Asp Val Phe Ile Ser Tyr Arg Arg Asn Ser Gly Ser Gln Leu Ala Ser
    530                 535                 540

Leu Leu Lys Val His Leu Gln Leu His Gly Phe Ser Val Phe Ile Asp
545                 550                 555                 560

Val Glu Lys Leu Glu Ala Gly Lys Phe Glu Asp Lys Leu Ile Gln Ser
                565                 570                 575

Val Met Gly Ala Arg Asn Phe Val Leu Val Leu Ser Pro Gly Ala Leu
            580                 585                 590

Asp Lys Cys Met Gln Asp His Asp Cys Lys Asp Trp Val His Lys Glu
        595                 600                 605

Ile Val Thr Ala Leu Ser Cys Gly Lys Asn Ile Val Pro Ile Ile Asp
    610                 615                 620

Gly Phe Glu Trp Pro Glu Pro Gln Val Leu Pro Glu Asp Met Gln Ala
625                 630                 635                 640

Val Leu Thr Phe Asn Gly Ile Lys Trp Ser His Glu Tyr Gln Glu Ala
                645                 650                 655

Thr Ile Glu Lys Ile Ile Arg Phe Leu Gln Gly Arg Ser Ser Arg Asp
            660                 665                 670

Ser Ser Ala Gly Ser Asp Thr Ser Leu Glu Gly Ala Ala Pro Met Gly
        675                 680                 685

Pro Thr
    690
```

<210> 3
<211> 28
<212> DNA
<213> Artificial Sequence

<220>
<223> Description of Artificial Sequence: primer 1

<400> 3
cgatgctagc atgccgttcc cgtttggg 28

<210> 4
<211> 28
<212> DNA
<213> Artificial Sequence

<220>
<223> Description of Artificial Sequence: primer 2

<400> 4
cgatccatgg ttaagcttct tgctgagc 28

<210> 5
<211> 496
<212> PRT
<213> Artificial Sequence

<220>
<223> Description of Artificial Sequence: Gal4-ANIC-BP-1 fusion protein

<400> 5

Met Lys Leu Leu Ser Ser Ile Glu Gln Ala Cys Asp Ile Cys Arg Leu
1 5 10 15

Lys Lys Leu Lys Cys Ser Lys Glu Lys Pro Lys Cys Ala Lys Cys Leu
20 25 30

Lys Asn Asn Trp Glu Cys Arg Tyr Ser Pro Lys Thr Lys Arg Ser Pro
35 40 45

Leu Thr Arg Ala His Leu Thr Glu Val Glu Ser Arg Leu Glu Arg Leu
50 55 60

Glu Gln Leu Phe Leu Leu Ile Phe Pro Arg Glu Asp Leu Asp Met Ile
65 70 75 80

Leu Lys Met Asp Ser Leu Gln Asp Ile Lys Ala Leu Leu Thr Gly Leu
85 90 95

Phe Val Gln Asp Asn Val Asn Lys Asp Ala Val Thr Asp Arg Leu Ala
100 105 110

Ser Val Glu Thr Asp Met Pro Leu Thr Leu Arg Gln His Arg Ile Ser
115 120 125

Ala Thr Ser Ser Ser Glu Glu Ser Ser Asn Lys Gly Gln Arg Gln Leu
130 135 140

Thr Val Ser Ser Arg Ser Thr Pro Gly Ala Ser Met Pro Phe Pro Phe
145 150 155 160

Gly Lys Ser His Lys Ser Pro Ala Asp Ile Val Lys Asn Leu Lys Glu
165 170 175

Ser Met Ala Val Leu Glu Lys Gln Asp Ile Ser Asp Lys Lys Ala Glu
180 185 190

Lys Ala Thr Glu Glu Val Ser Lys Asn Leu Val Ala Met Lys Glu Ile
195 200 205

Leu Tyr Gly Thr Asn Glu Lys Glu Pro Gln Thr Glu Ala Val Ala Gln
210 215 220

Leu Ala Gln Glu Leu Tyr Asn Ser Gly Leu Leu Ser Thr Leu Val Ala
225 230 235 240

Asp Leu Gln Leu Ile Asp Phe Glu Gly Lys Lys Asp Val Ala Gln Ile
245 250 255

Phe Asn Asn Ile Leu Arg Arg Gln Ile Gly Thr Arg Thr Pro Thr Val
260 265 270

Glu Tyr Ile Cys Thr Gln Gln Asn Ile Leu Phe Met Leu Leu Lys Gly
275 280 285

Tyr Glu Ser Pro Glu Ile Ala Leu Asn Cys Gly Ile Met Leu Arg Glu
290 295 300

Cys Ile Arg His Glu Pro Leu Ala Lys Ile Ile Leu Trp Ser Glu Gln
305 310 315 320

Phe Tyr Asp Phe Phe Arg Tyr Val Glu Met Ser Thr Phe Asp Ile Ala
325 330 335

Ser Asp Ala Phe Ala Thr Phe Lys Asp Leu Leu Thr Arg His Lys Leu
340 345 350

Leu Ser Ala Glu Phe Leu Glu Gln His Tyr Asp Arg Phe Phe Ser Glu
355 360 365

Tyr Glu Lys Leu Leu His Ser Glu Asn Tyr Val Thr Lys Arg Gln Ser
370 375 380

Leu Lys Leu Leu Gly Glu Leu Leu Leu Asp Arg His Asn Phe Thr Ile
385 390 395 400

Met Thr Lys Tyr Ile Ser Lys Pro Glu Asn Leu Lys Leu Met Met Asn
405 410 415

Leu Leu Arg Asp Lys Ser Arg Asn Ile Gln Phe Glu Ala Phe His Val
420 425 430

Phe Lys Val Phe Val Ala Asn Pro Asn Lys Thr Gln Pro Ile Leu Asp
435 440 445

Ile Leu Leu Lys Asn Gln Ala Lys Leu Ile Glu Phe Leu Ser Lys Phe
450 455 460

Gln Asn Asp Arg Thr Glu Asp Glu Gln Phe Asn Asp Glu Lys Thr Tyr

465 470 475 480

Leu Val Lys Gln Ile Arg Asp Leu Lys Arg Pro Ala Gln Gln Glu Ala
485 490 495

<210> 6
<211> 552
<212> PRT
<213> Artificial Sequence

<220>
<223> Description of Artificial Sequence: LexA-ANIC-BP-1 fusion protein

<400> 6

```
Met Lys Ala Leu Thr Ala Arg Gln Gln Glu Val Phe Asp Leu Ile Arg
 1               5                  10                  15

Asp His Ile Ser Gln Thr Gly Met Pro Pro Thr Arg Ala Glu Ile Ala
            20                  25                  30

Gln Arg Leu Gly Phe Arg Ser Pro Asn Ala Ala Glu Glu His Leu Lys
        35                  40                  45

Ala Leu Ala Arg Lys Gly Val Ile Glu Ile Val Ser Gly Ala Ser Arg
    50                  55                  60

Gly Ile Arg Leu Leu Gln Glu Glu Glu Glu Gly Leu Pro Leu Val Gly
65                  70                  75                  80

Arg Val Ala Ala Gly Glu Pro Leu Leu Ala Gln Gln His Ile Glu Gly
                85                  90                  95

His Tyr Gln Val Asp Pro Ser Leu Phe Lys Pro Asn Ala Asp Phe Leu
            100                 105                 110

Leu Arg Val Ser Gly Met Ser Met Lys Asp Ile Gly Ile Met Asp Gly
        115                 120                 125

Asp Leu Leu Ala Val His Lys Thr Gln Asp Val Arg Asn Gly Gln Val
    130                 135                 140

Val Val Ala Arg Ile Asp Asp Glu Val Thr Val Lys Arg Leu Lys Lys
145                 150                 155                 160

Gln Gly Asn Lys Val Glu Leu Leu Pro Glu Asn Ser Glu Phe Lys Pro
                165                 170                 175

Ile Val Val Asp Leu Arg Gln Gln Ser Phe Thr Ile Glu Gly Leu Ala
            180                 185                 190

Val Gly Val Ile Arg Asn Gly Asp Trp Leu Glu Phe Arg Ser Thr Pro
        195                 200                 205

Gly Ala Ser Met Pro Phe Pro Phe Gly Lys Ser His Lys Ser Pro Ala
    210                 215                 220
```

```
Asp Ile Val Lys Asn Leu Lys Glu Ser Met Ala Val Leu Glu Lys Gln
225                 230                 235                 240

Asp Ile Ser Asp Lys Lys Ala Glu Lys Ala Thr Glu Glu Val Ser Lys
                245                 250                 255

Asn Leu Val Ala Met Lys Glu Ile Leu Tyr Gly Thr Asn Glu Lys Glu
            260                 265                 270

Pro Gln Thr Glu Ala Val Ala Gln Leu Ala Gln Glu Leu Tyr Asn Ser
        275                 280                 285

Gly Leu Leu Ser Thr Leu Val Ala Asp Leu Gln Leu Ile Asp Phe Glu
    290                 295                 300

Gly Lys Lys Asp Val Ala Gln Ile Phe Asn Asn Ile Leu Arg Arg Gln
305                 310                 315                 320

Ile Gly Thr Arg Thr Pro Thr Val Glu Tyr Ile Cys Thr Gln Gln Asn
                325                 330                 335

Ile Leu Phe Met Leu Leu Lys Gly Tyr Glu Ser Pro Glu Ile Ala Leu
            340                 345                 350

Asn Cys Gly Ile Met Leu Arg Glu Cys Ile Arg His Glu Pro Leu Ala
        355                 360                 365

Lys Ile Ile Leu Trp Ser Glu Gln Phe Tyr Asp Phe Phe Arg Tyr Val
    370                 375                 380

Glu Met Ser Thr Phe Asp Ile Ala Ser Asp Ala Phe Ala Thr Phe Lys
385                 390                 395                 400

Asp Leu Leu Thr Arg His Lys Leu Leu Ser Ala Glu Phe Leu Glu Gln
                405                 410                 415

His Tyr Asp Arg Phe Phe Ser Glu Tyr Glu Lys Leu Leu His Ser Glu
            420                 425                 430

Asn Tyr Val Thr Lys Arg Gln Ser Leu Lys Leu Leu Gly Glu Leu Leu
        435                 440                 445

Leu Asp Arg His Asn Phe Thr Ile Met Thr Lys Tyr Ile Ser Lys Pro
    450                 455                 460

Glu Asn Leu Lys Leu Met Met Asn Leu Leu Arg Asp Lys Ser Arg Asn
465                 470                 475                 480

Ile Gln Phe Glu Ala Phe His Val Phe Lys Val Phe Val Ala Asn Pro
                485                 490                 495

Asn Lys Thr Gln Pro Ile Leu Asp Ile Leu Leu Lys Asn Gln Ala Lys
            500                 505                 510

Leu Ile Glu Phe Leu Ser Lys Phe Gln Asn Asp Arg Thr Glu Asp Glu
        515                 520                 525

Gln Phe Asn Asp Glu Lys Thr Tyr Leu Val Lys Gln Ile Arg Asp Leu
    530                 535                 540

Lys Arg Pro Ala Gln Gln Glu Ala
```

545 550

<210> 7
<211> 29
<212> DNA
<213> Artificial Sequence

<220>
<223> Description of Artificial Sequence: primer 3

<400> 7
aattccaggt cgacctcgag gcggccgct 29

<210> 8
<211> 29
<212> DNA
<213> Artificial Sequence

<220>
<223> Description of Artificial Sequence: primer 4

<400> 8
tcgaaccggc cgcctcgagg tcgacctgg 29

## Description

### Field of the Invention

This invention relates to newly identified polypeptides and polynucleotides encoding such polypeptides sometimes hereinafter referred to as "ANIC-BP-1 ligand", to their use in diagnosis and in identifying compounds that may be agonists, antagonists that are potentially useful in therapy, and to production of such polypeptides and polynucleotides.

### Background of the Invention

The drug discovery process is currently undergoing a fundamental revolution as it embraces "functional genomics", that is, high throughput genome- or gene-based biology. This approach as a means to identify genes and gene products as therapeutic targets is rapidly superceding earlier approaches based on "positional cloning". A phenotype, that is a biological function or genetic disease, would be identified and this would then be tracked back to the responsible gene, based on its genetic map position.
Functional genomics relies heavily on high-throughput DNA sequencing technologies and the various tools of bioinformatics to identify gene sequences of potential interest from the many molecular biology databases now available. There is a continuing need to identify and characterise further genes and their related polypeptides/proteins, as targets for drug discovery.

### Summary of the Invention

The present invention relates to ANIC-BP-1 ligand, in particular ANIC-BP-1 ligand polypeptides and ANIC-BP-1 ligand polynucleotides, recombinant materials and methods for their production. Such polypeptides and polynucleotides are of interest in relation to methods of treatment of certain diseases, including, but not limited to, stroke, head trauma, multiple sclerosis, Parkinson's disease, Alzheimer's disease, spinal cord injury, hereinafter referred to as " diseases of the invention". In a further aspect, the invention relates to methods for identifying agonists and antagonists (e.g., inhibitors) using the materials provided by the invention, and treating conditions associated with ANIC-BP-1 ligand imbalance with the identified compounds. In a still further aspect, the invention relates to diagnostic assays for detecting diseases associated with inappropriate ANIC-BP-1 ligand activity or levels.

### Description of the Invention

In a first aspect, the present invention relates to ANIC-BP-1 ligand polypeptides. Such polypeptides include:

(a) a polypeptide encoded by a polynucleotide comprising the sequence of SEQ ID NO:1;

(b) a polypeptide comprising a polypeptide sequence having at least 95%, 96%, 97%, 98%, or 99% identity to the polypeptide sequence of SEQ ID NO:2;

(c) a polypeptide comprising the polypeptide sequence of SEQ ID NO:2;

(d) a polypeptide having at least 95%, 96%, 97%, 98%, or 99% identity to the polypeptide sequence of SEQ ID NO:2;

(e) the polypeptide sequence of SEQ ID NO:2; and

(f) a polypeptide having or comprising a polypeptide sequence that has an Identity Index of 0.95, 0.96, 0.97, 0.98, or 0.99 compared to the polypeptide sequence of SEQ ID NO:2.

Polypeptides of the present invention are believed to be members of the sterile alpha motif containing protein family family of polypeptides. They are therefore of interest because three described gene members of the ANIC-BP family, called ANIC-BP-1, ANIC-BP-2 and ANIC-BP-1B have been identified recently. ANIC-BP-1 was found up-regulated in a rat model of head trauma as discovered with the technique of mRNA differential display The.
The biological properties of the ANIC-BP-1 ligand are hereinafter referred to as "biological activity of ANIC-BP-1 ligand" or "ANIC-BP-1 ligand activity". Preferably, a polypeptide of the present invention exhibits at least one biological activity of ANIC-BP-1 ligand.
Fragments of the polypeptides of the invention may be employed for producing the corresponding full-length polypeptide by peptide synthesis; therefore, these variants may be employed as intermediates for producing the full-length polypeptides of the invention. The polypeptides of the present invention may be in the form of the "mature" protein or may be a part of a larger protein such as a precursor or a fusion protein. It is often advantageous to include an additional amino acid sequence that contains secretory or leader sequences, pro-sequences, sequences that aid in purification, for instance multiple histidine residues, or an additional sequence for stability during recombinant production.

Polypeptides of the present invention can be prepared in any suitable manner, for instance by isolation form naturally occurring sources, from genetically engineered host cells comprising expression systems (vide *infra*) or by chemical synthesis, using for instance automated peptide synthesisers, or a combination of such methods.. Means for preparing such polypeptides are well understood in the art.

In a further aspect, the present invention relates to ANIC-BP-1 ligand polynucleotides. Such polynucleotides include:

(a) a polynucleotide comprising a polynucleotide sequence having at least 95%, 96%, 97%, 98%, or 99% identity to the polynucleotide sequence of SEQ ID NO:1;

(b) a polynucleotide comprising the polynucleotide of SEQ ID NO:1;

(c) a polynucleotide having at least 95%, 96%, 97%, 98%, or 99% identity to the polynucleotide of SEQ ID NO:1;

(d) the polynucleotide of SEQ ID NO:1;

(e) a polynucleotide comprising a polynucleotide sequence encoding a polypeptide sequence having at least 95%, 96%, 97%, 98%, or 99% identity to the polypeptide sequence of SEQ ID NO:2;

(f) a polynucleotide comprising a polynucleotide sequence encoding the polypeptide of SEQ ID NO:2;

(g) a polynucleotide having a polynucleotide sequence encoding a polypeptide sequence having at least 95%, 96%, 97%, 98%, or 99% identity to the polypeptide sequence of SEQ ID NO:2;

(h) a polynucleotide encoding the polypeptide of SEQ ID NO:2;

(i) a polynucleotide having or comprising a polynucleotide sequence that has an Identity Index of 0.95, 0.96, 0.97, 0.98, or 0.99 compared to the polynucleotide sequence of SEQ ID NO:1;

(j) a polynucleotide having or comprising a polynucleotide sequence encoding a polypeptide sequence that has an Identity Index of 0.95, 0.96, 0.97, 0.98, or 0.99 compared to the polypeptide sequence of SEQ ID NO:2; and polynucleotides that are complementary to above mentioned polynucleotides, over the entire length thereof.

In a further aspect, the present invention provides polynucleotides that are RNA transcripts of the DNA sequences of the present invention. Accordingly, there is provided an RNA polynucleotide that:

(a) comprises an RNA transcript of the DNA sequence encoding the polypeptide of SEQ ID NO:2;

(b) is the RNA transcript of the DNA sequence encoding the polypeptide of SEQ ID NO:2;

(c) comprises an RNA transcript of the DNA sequence of SEQ ID NO:1; or

(d) is the RNA transcript of the DNA sequence of SEQ ID NO:1;

and RNA polynucleotides that are complementary thereto.

The polynucleotide sequence of SEQ ID NO: 1 shows homology with AB011096 (KIAA0524; Nagase T. et al., DNA Research 5, 31 - 39; 1998). The polynucleotide sequence of SEQ ID NO:1 is a cDNA sequence that encodes the polypeptide of SEQ ID NO:2. The polynucleotide sequence encoding the polypeptide of SEQ ID NO:2 may be identical to the polypeptide encoding sequence of SEQ ID NO:1 or it may be a sequence other than SEQ ID NO:1, which, as a result of the redundancy (degeneracy) of the genetic code, also encodes the polypeptide of SEQ ID NO:2. The polypeptide of the SEQ ID NO:2 is related to other proteins of the sterile alpha motif containing protein family family, having homology and/or structural similarity with GI-3043572 (KIAA0524; Nagase T. et al., DNA Research 5, 31 - 39; 1998).

Preferred polypeptides and polynucleotides of the present invention are expected to have, *inter alia,* similar biological functions/properties to their homologous polypeptides and polynucleotides. Furthermore, preferred polypeptides and polynucleotides of the present invention have at least one ANIC-BP ligand activity.

Polynucleotides of the present, invention may be obtained using standard cloning and screening techniques from a cDNA library derived from mRNA in cells of human bone, brain, breast, colon, germ cell, heart, ovary, pancreas, parathyroid, placenta, spleen, tonsil, uterus and colon, (see for instance, Sambrook *et al.*, Molecular Cloning: A Laboratory Manual, 2nd Ed., Cold Spring Harbor Laboratory Press, Cold Spring Harbor, N.Y. (1989)). Polynucleotides of the invention can also be obtained from natural sources such as genomic DNA libraries or can be synthesized using well known and commercially available techniques.

When polynucleotides of the present invention are used for the recombinant production of polypeptides of the present invention, the polynucleotide may include the coding sequence for the mature polypeptide, by itself, or the coding sequence for the mature polypeptide in reading frame with other coding sequences, such as those encoding a leader or secretory sequence, a pre-, or pro- or prepro- protein sequence, or other fusion peptide portions. For example, a marker sequence that facilitates purification of the fused polypeptide can be encoded. In certain preferred embodiments of this aspect of the invention, the marker sequence is a hexa-histidine peptide, as provided in the pQE vector (Qiagen, Inc.) and described in Gentz *et al.,* Proc Natl Acad Sci USA (1989) 86:821-824, or is an HA tag. The polynucleotide may also contain non-coding 5' and 3' sequences, such as transcribed, non-translated sequences, splicing and polyadenylation signals, ribosome binding sites and sequences that stabilize mRNA.

Polynucleotides that are identical, or have sufficient identity to a polynucleotide sequence of SEQ ID NO:1, may be used as hybridization probes for cDNA and genomic DNA or as primers for a nucleic acid amplification reaction (for instance, PCR). Such probes and primers may be used to isolate full-length cDNAs and genomic clones encoding polypeptides of the present invention and to isolate cDNA and genomic clones of other genes (including genes encoding paralogs from human sources and orthologs and paralogs from species other than human) that have a high sequence similarity to SEQ ID NO:1, typically at least 95% identity. Preferred probes and primers will generally comprise at least 15 nucleotides, preferably, at least 30 nucleotides and may have at least 50, if not at least 100 nucleotides. Particularly preferred probes will have between 30 and 50 nucleotides. Particularly preferred primers will have between 20 and 25 nucleotides.

A polynucleotide encoding a polypeptide of the present invention, including homologs from species other than human, may be obtained by a process comprising the steps of screening a library under stringent hybridization conditions with a labeled probe having the sequence of SEQ ID NO: 1 or a fragment thereof, preferably of at least 15 nucleotides; and isolating full-length cDNA and genomic clones containing said polynucleotide sequence. Such hybridization techniques are well known to the skilled artisan. Preferred stringent hybridization conditions include overnight incubation at 42°C in a solution comprising: 50% formamide, 5xSSC (150mM NaCl, 15mM trisodium citrate), 50 mM sodium phosphate (pH7.6), 5x Denhardt's solution; 10 % dextran sulfate, and 20 microgram/ml denatured, sheared salmon sperm DNA; followed by washing the filters in 0.1x SSC at about 65°C.

The skilled artisan will appreciate that, in many cases, an isolated cDNA sequence will be incomplete, in that the region coding for the polypeptide does not extend all the way through to the 5' terminus. This is a consequence of reverse transcriptase, an enzyme with inherently low "processivity" (a measure of the ability of the enzyme to remain attached to the template during the polymerisation reaction), failing to complete a DNA copy of the mRNA template during first strand cDNA synthesis.

There are several methods available and well known to those skilled in the art to obtain full-length cDNAs, or extend short cDNAs, for example those based on the method of Rapid Amplification of cDNA ends (RACE) (see, for example, Frohman et al., Proc Nat Acad Sci USA 85, 8998-9002, 1988). Recent modifications of the technique, exemplified by the Marathon (trade mark) technology (Clontech Laboratories Inc.) for example, have significantly simplified the search for longer cDNAs. In the Marathon (trade mark) technology, cDNAs have been prepared from mRNA extracted from a chosen tissue and an 'adaptor' sequence ligated onto each end. Nucleic acid amplification (PCR) is then carried out to amplify the "missing" 5' end of the cDNA using a combination of gene specific and adaptor specific oligonucleotide primers. The PCR reaction is then repeated using 'nested' primers, that is, primers designed to anneal within the amplified product (typically an adaptor specific primer that anneals further 3' in the adaptor sequence and a gene specific primer that anneals further 5' in the known gene sequence). The products of this reaction can then be analysed by DNA sequencing and a full-length cDNA constructed either by joining the product directly to the existing cDNA to give a complete sequence, or carrying out a separate full-length PCR using the new sequence information for the design of the 5' primer.

Recombinant polypeptides of the present invention may be prepared by processes well known in the art from genetically engineered host cells comprising expression systems. Accordingly, in a further aspect, the present invention relates to expression systems comprising a polynucleotide or polynucleotides of the present invention, to host cells which are genetically engineered with such expression sytems and to the production of polypeptides of the invention by recombinant techniques. Cell-free translation systems can also be employed to produce such proteins using RNAs derived from the DNA constructs of the present invention.

For recombinant production, host cells can be genetically engineered to incorporate expression systems or portions thereof for polynucleotides of the present invention. Polynucleotides may be introduced into host cells by methods described in many standard laboratory manuals, such as Davis et al., Basic Methods in Molecular Biology (1986) and Sambrook *et al.(ibid).* Preferred methods of introducing polynucleotides into host cells include, for instance, calcium phosphate transfection, DEAE-dextran mediated transfection, transvection, microinjection, cationic lipid-mediated transfection, electroporation, transduction, scrape loading, ballistic introduction or infection.

Representative examples of appropriate hosts include bacterial cells, such as *Streptococci, Staphylococci, E. coli, Streptomyces* and *Bacillus subtilis* cells; fungal cells, such as yeast cells and *Aspergillus* cells; insect cells such as *Drosophila* S2 and *Spodoptera* Sf9 cells; animal cells such as CHO, COS, HeLa, C127, 3T3, BHK, HEK 293 and Bowes melanoma cells; and plant cells.

A great variety of expression systems can be used, for instance, chromosomal, episomal and virus-derived systems, e.g., vectors derived from bacterial plasmids, from bacteriophage, from transposons, from yeast episomes, from insertion elements, from yeast chromosomal elements, from viruses such as baculoviruses, papova viruses, such as SV40, vaccinia viruses, adenoviruses, fowl pox viruses, pseudorabies viruses and retroviruses, and vectors derived from combinations thereof, such as those derived from plasmid and bacteriophage genetic elements, such as cosmids and phagemids. The expression systems may contain control regions that regulate as well as engender expression. Generally, any system or vector that is able to maintain, propagate or express a polynucleotide to produce a polypeptide in a host may be used. The appropriate polynucleotide sequence may be inserted into an expression system by any of a variety of well-known and routine techniques, such as, for example, those set forth in Sambrook *et al., (ibid).* Appropriate secretion signals may be incorporated into the desired polypeptide to allow secretion of the translated protein into the lumen of the endoplasmic reticulum, the periplasmic space or the extracellular environment. These signals may be endogenous to the polypeptide or they may be heterologous signals.

If a polypeptide of the present invention is to be expressed for use in screening assays, it is generally preferred that the polypeptide be produced at the surface of the cell. In this event, the cells may be harvested prior to use in the screening assay. If the polypeptide is secreted into the medium, the medium can be recovered in order to recover and purify the polypeptide. If produced intracellularly, the cells must first be lysed before the polypeptide is recovered.

Polypeptides of the present invention can be recovered and purified from recombinant cell cultures by well-known methods including ammonium sulfate or ethanol precipitation, acid extraction, anion or cation exchange chromatography, phosphocellulose chromatography, hydrophobic interaction chromatography, affinity chromatography, hydroxylapatite chromatography and lectin chromatography. Most preferably, high performance liquid chromatography is employed for purification. Well known techniques for refolding proteins may be employed to regenerate active conformation when the polypeptide is denatured during intracellular synthesis, isolation and/or purification.

Polynucleotides of the present invention may be used as diagnostic reagents, through detecting mutations in the associated gene. Detection of a mutated form of the gene characterised by the polynucleotide of SEQ ID NO:1 in the cDNA or genomic sequence and which is associated with a dysfunction will provide a diagnostic tool that can add to, or define, a diagnosis of a disease, or susceptibility to a disease, which results from under-expression, over-expression or altered spatial or temporal expression of the gene. Individuals carrying mutations in the gene may be detected at the DNA level by a variety of techniques well known in the art.

Nucleic acids for diagnosis may be obtained from a subject's cells, such as from blood, urine, saliva, tissue biopsy or autopsy material. The genomic DNA may be used directly for detection or it may be amplified enzymatically by using PCR, preferably RT-PCR, or other amplification techniques prior to analysis. RNA or cDNA may also be used in similar fashion. Deletions and insertions can be detected by a change in size of the amplified product in comparison to the normal genotype. Point mutations can be identified by hybridizing amplified DNA to labeled ANIC-BP ligand nucleotide sequences. Perfectly matched sequences can be distinguished from mismatched duplexes by RNase digestion or by differences in melting temperatures. DNA sequence difference may also be detected by alterations in the electrophoretic mobility of DNA fragments in gels, with or without denaturing agents, or by direct DNA sequencing (see, for instance, Myers *et al*., Science (1985) 230:1242). Sequence changes at specific locations may also be revealed by nuclease protection assays, such as RNase and S1 protection or the chemical cleavage method (see Cotton *et al.,* Proc Natl Acad Sci USA (1985) 85: 4397-4401).

An array of oligonucleotides probes comprising ANIC-BP ligand polynucleotide sequence or fragments thereof can be constructed to conduct efficient screening of *e.g*., genetic mutations. Such arrays are preferably high density arrays or grids. Array technology methods are well known and have general applicability and can be used to address a variety of questions in molecular genetics including gene expression, genetic linkage, and genetic variability, see, for example, M.Chee et al., Science, 274, 610-613 (1996) and other references cited therein.

Detection of abnormally decreased or increased levels of polypeptide or mRNA expression may also be used for diagnosing or determining susceptibility of a subject to a disease of the invention. Decreased or increased expression can be measured at the RNA level using any of the methods well known in the art for the quantitation of polynucleotides, such as, for example, nucleic acid amplification, for instance PCR, RT-PCR, RNase protection, Northern blotting and other hybridization methods. Assay techniques that can be used to determine levels of a protein, such as a polypeptide of the present invention, in a sample derived from a host are well-known to those of skill in the art. Such assay methods include radioimmunoassays, competitive-binding assays, Western Blot analysis and ELISA assays.

Thus in another aspect, the present invention discloses to a diagonostic kit comprising:

(a) a polynucleotide of the present invention, preferably the nucleotide sequence of SEQ ID NO: 1, or an RNA transcript thereof;

(b) a nucleotide sequence complementary to that of (a);

(c) a polypeptide of the present invention, preferably the polypeptide of SEQ ID NO:2.

It will be appreciated that in any such kit, (a), (b), or (c) may comprise a substantial component. Such a kit will be of use in diagnosing a disease or susceptibility to a disease, particularly diseases of the invention, amongst others.

The polynucleotide sequences of the present invention are valuable for chromosome localisation studies. The sequence is specifically targeted to, and can hybridize with, a particular location on an individual human chromosome. The mapping of relevant sequences to chromosomes according to the present invention is an important first step in correlating those sequences with gene associated disease. Once a sequence has been mapped to a precise chromosomal location, the physical position of the sequence on the chromosome can be correlated with genetic map data. Such data are found in, for example, V. McKusick, Mendelian Inheritance in Man (available on-line through Johns Hopkins University Welch Medical Library). The relationship between genes and diseases that have been mapped to the same chromosomal region are then identified through linkage analysis (co-inheritance of physically adjacent genes). Precise human chromosomal localisations for a genomic sequence (gene fragment etc.) can be determined using Radiation Hybrid (RH) Mapping (Walter, M. Spillett, D., Thomas, P., Weissenbach, J., and Goodfellow, P., (1994) A method for constructing radiation hybrid maps of whole genomes, Nature Genetics 7, 22-28). A number of RH panels are available from Research Genetics (Huntsville, AL, USA) e.g. the GeneBridge4 RH panel (Hum Mol Genet 1996 Mar;5(3):339-46 A radiation hybrid map of the human genome. Gyapay G, Schmitt K, Fizames C, Jones H, Vega-Czarny N, Spillett D, Muselet D, Prud'Homme JF, Dib C, Auffray C, Morissette J, Weissenbach J, Goodfellow PN). To determine the chromosomal location of a gene using this panel, 93 PCRs are performed using primers designed from the gene of interest on RH DNAs. Each of these DNAs contains random human genomic fragments maintained in a hamster background (human / hamster hybrid cell lines). These PCRs result in 93 scores indicating the presence or absence of the PCR product of the gene of interest. These scores are compared with scores created using PCR products from genomic sequences of known location. This comparison is conducted at http://www.genome.wi.mit.edu/. The gene of the present invention maps to human chromosome Chr. 17 (D17S922-D17S798).

The polynucleotide sequences of the present invention are also valuable tools for tissue expression studies. Such studies allow the determination of expression patterns of polynucleotides of the present invention which may give an indication as to the expression patterns of the encoded polypeptides in tissues, by detecting the mRNAs that encode them. The techniques used are well known in the art and include in situ hydridisation techniques to clones arrayed on a grid, such as cDNA microarray hybridisation (Schena *et al,* Science, 270, 467-470, 1995 and Shalon *et al,* Genome Res, 6, 639-645, 1996) and nucleotide amplification techniques such as PCR. A preferred method uses the TAQMAN (Trade mark) technology available from Perkin Elmer. Results from these studies can provide an indication of the normal function of the polypeptide in the organism. In addition, comparative studies of the normal expression pattern of mRNAs with that of mRNAs encoded by an alternative form of the same gene (for example, one having an alteration in polypeptide coding potential or a regulatory mutation) can provide valuable insights into the role of the polypeptides of the present invention, or that of inappropriate expression thereof in disease. Such inappropriate expression may be of a temporal, spatial or simply quantitative nature.

The polypeptides of the present invention are expressed in bone, brain, breast, colon, germ cell, heart, ovary, pancreas, parathyroid, placenta, spleen, tonsil, uterus, colon.

A further aspect of the present invention discloses antibodies. The polypeptides of the invention or their fragments, or cells expressing them, can be used as immunogens to produce antibodies that are immunospecific for polypeptides of the present invention. The term "immunospecific" means that the antibodies have substantially greater affinity for the polypeptides of the invention than their affinity for other related polypeptides in the prior art.

Antibodies generated against polypeptides of the present invention may be obtained by administering the polypeptides or epitope-bearing fragments, or cells to an animal, preferably a non-human animal, using routine protocols. For preparation of monoclonal antibodies, any technique which provides antibodies produced by continuous cell line cultures can be used. Examples include the hybridoma technique (Kohler, G. and Milstein, C., Nature (1975) 256: 495-497), the trioma technique, the human B-cell hybridoma technique (Kozbor *et al.,* Immunology Today (1983) 4:72) and the EBV-hybridoma technique (Cole *et al.,* Monoclonal Antibodies and Cancer Therapy, 77-96, Alan R. Liss, Inc., 1985).

Techniques for the production of single chain antibodies, such as those described in U.S. Patent No. 4,946,778, can also be adapted to produce single chain antibodies to polypeptides of this invention. Also, transgenic mice, or other organisms, including other mammals, may be used to express humanized antibodies.

The above-described antibodies may be employed to isolate or to identify clones expressing the polypeptide or to purify the polypeptides by affinity chromatography. Antibodies against polypeptides of the present invention may also be employed to treat diseases of the invention, amongst others.

Polypeptides and polynucleotides of the present invention may also be used as vaccines. Accordingly, in a further aspect, the present invention relates to a method for inducing an immunological response in a mammal that comprises inoculating the mammal with a polypeptide of the present invention, adequate to produce antibody and/or T cell immune response, including, for example, cytokine-producing T cells or cytotoxic T cells, to protect said animal from disease, whether that disease is already established within the individual or not. An immunological response in a mammal may also be induced by a method comprises delivering a polypeptide of the present invention *via* a vector directing expression of the polynucleotide and coding for the polypeptide *in vivo* in order to induce such an immunological response to produce antibody to protect said animal from diseases of the invention. One way of administering the vector is by accelerating it into the desired cells as a coating on particles or otherwise. Such nucleic acid vector may comprise DNA, RNA, a modified nucleic acid, or a DNA/RNA hybrid. For use a vaccine, a polypeptide or a nucleic acid vector will be normally provided as a vaccine formulation (composition). The formulation may further comprise a suitable carrier. Since a polypeptide may be broken down in the stomach, it is preferably administered parenterally (for instance, subcutaneous, intramuscular, intravenous, or intradermal injection). Formulations suitable for parenteral administration include aqueous and non-aqueous sterile injection solutions that may contain anti-oxidants, buffers, bacteriostats and solutes that render the formulation instonic with the blood of the recipient; and aqueous and non-aqueous sterile suspensions that may include suspending agents or thickening agents. The formulations may be presented in unit-dose or multi-dose containers, for example, sealed ampoules and vials and may be stored in a freeze-dried condition requiring only the addition of the sterile liquid carrier immediately prior to use. The vaccine formulation may also include adjuvant systems for enhancing the immunogenicity of the formulation, such as oil-in water systems and other systems known in the art. The dosage will depend on the specific activity of the vaccine and can be readily determined by routine experimentation.

Polypeptides of the present invention have one or more biological functions that are of relevance in one or more disease states, in particular the diseases of the invention hereinbefore mentioned. It is therefore useful to to identify compounds that stimulate or inhibit the function or level of the polypeptide. Accordingly, in a further aspect, the present invention provides for a method of screening compounds to identify those that stimulate or inhibit the function or level of the polypeptide. Such methods identify agonists or antagonists that may be employed for therapeutic and prophylactic purposes for such diseases of the invention as hereinbefore mentioned. Compounds may be identified from a variety of sources, for example, cells, cell-free preparations, chemical libraries, collections of chemical compounds, and natural product mixtures. Such agonists or antagonists so-identified may be natural or modified substrates, ligands, receptors, enzymes, etc., as the case may be, of the polypeptide; a structural or functional mimetic thereof (see Coligan *et al.*, Current Protocols in Immunology 1 (2):Chapter 5 (1991)) or a small molecule.

The screening method may simply measure the binding of a candidate compound to the polypeptide, or to cells or membranes bearing the polypeptide, or a fusion protein thereof, by means of a label directly or indirectly associated with the candidate compound. Alternatively, the screening method may involve measuring or detecting (qualitatively or quantitatively) the competitive binding of a candidate compound to the polypeptide against a labeled competitor (e.g. agonist or antagonist). Further, these screening methods may test whether the candidate compound results in a signal generated by activation or inhibition of the polypeptide, using detection systems appropriate to the cells bearing the polypeptide. Inhibitors of activation are generally assayed in the presence of a known agonist and the effect on activation by the agonist by the presence of the candidate compound is observed. Further, the screening methods may simply comprise the steps of mixing a candidate compound with a solution containing a polypeptide of the present invention, to form a mixture, measuring a ANIC-BP ligand activity in the mixture, and comparing the ANIC-BP ligand activity of the mixture to a control mixture which contains no candidate compound.

Polypeptides of the present invention may be employed in conventional low capacity screening methods and also in high-throughput screening (HTS) formats. Such HTS formats include not only the well-established use of 96- and, more recently, 384-well micotiter plates but also emerging methods such as the nanowell method described by Schullek et al, Anal Biochem., 246, 20-29, (1997).

Fusion proteins, such as those made from Fc portion and ANIC-BP ligand polypeptide, as hereinbefore described, can also be used for high-throughput screening assays to identify antagonists for the polypeptide of the present invention (see D. Bennett *et al.,* J Mol Recognition, 8:52-58 (1995); and K. Johanson *et al.,* J Biol Chem, 270(16): 9459-9471 (1995)).

Screening techniques

The polynucleotides, polypeptides and antibodies to the polypeptide of the present invention may also be used to configure screening methods for detecting the effect of added compounds on the production of mRNA and polypeptide in cells. For example, an ELISA assay may be constructed for measuring secreted or cell associated levels of polypeptide using monoclonal and polyclonal antibodies by standard methods known in the art. This can be used to discover agents that may inhibit or enhance the production of polypeptide (also called antagonist or agonist, respectively) from suitably manipulated cells or tissues.

A polypeptide of the present invention may be used to identify membrane bound or soluble receptors, if any, through standard receptor binding techniques known in the art. These include, but are not limited to, ligand binding and crosslinking assays in which the polypeptide is labeled with a radioactive isotope (for instance, $^{125}I$), chemically modified (for instance, biotinylated), or fused to a peptide sequence suitable for detection or purification, and incubated with a source of the putative receptor (cells, cell membranes, cell supernatants, tissue extracts, bodily fluids). Other methods include biophysical techniques such as surface plasmon resonance and spectroscopy. These screening methods may also be used to identify agonists and antagonists of the polypeptide that compete with the binding of the polypeptide to its receptors, if any. Standard methods for conducting such assays are well understood in the art.

Examples of antagonists of polypeptides of the present invention include antibodies or, in some cases, oligonucleotides or proteins that are closely related to the ligands, substrates, receptors, enzymes, etc., as the case may be, of the polypeptide, e.g., a fragment of the ligands, substrates, receptors, enzymes, etc.; or a small molecule that bind to the polypeptide of the present invention but do not elicit a response, so that the activity of the polypeptide is prevented.

Screening methods may also involve the use of transgenic technology and ANIC-BP-1 ligand gene. The art of constructing transgenic animals is well established. For example, the ANIC-BP-1 ligand gene may be introduced through microinjection into the male pronucleus of fertilized oocytes, retroviral transfer into pre- or post-implantation embryos, or injection of genetically modified, such as by electroporation, embryonic stem cells into host blastocysts. Particularly useful transgenic animals are so-called "knock-in" animals in which an animal gene is replaced by the human equivalent within the genome of that animal. Knock-in transgenic animals are useful in the drug discovery process, for target validation, where the compound is specific for the human target. Other useful transgenic animals are so-called "knock-out" animals in which the expression of the animal ortholog of a polypeptide of the present invention and encoded by an endogenous DNA sequence in a cell is partially or completely annulled. The gene knock-out may be targeted to specific cells or tissues, may occur only in certain cells or tissues as a consequence of the limitations of the technology, or may occur in all, or substantially all, cells in the animal. Transgenic animal technology also offers a whole animal expression-cloning system in which introduced genes are expressed to give large amounts of polypeptides of the present invention.

Screening kits for use in the above described methods are disclosed. Such screening kits comprise:

(a) a polypeptide of the present invention;

(b) a recombinant cell expressing a polypeptide of the present invention;

(c) a cell membrane expressing a polypeptide of the present invention; or

(d) an antibody to a polypeptide of the present invention;

which polypeptide is preferably that of SEQ ID NO:2.

It will be appreciated that in any such kit, (a), (b), (c) or (d) may comprise a substantial component.

**Glossary**

The following definitions are provided to facilitate understanding of certain terms used frequently hereinbefore.

"Antibodies" as used herein includes polyclonal and monoclonal antibodies, chimeric, single chain, and humanized antibodies, as well as Fab fragments, including the products of an

Fab or other immunoglobulin expression library.

"Isolated" means altered "by the hand of man" from its natural state, *i.e.,* if it occurs in nature, it has been changed or removed from its original environment, or both. For example, a polynucleotide or a polypeptide naturally present in a living organism is not "isolated," but the same polynucleotide or polypeptide separated from the coexisting materials of its natural state is "isolated", as the term is employed herein. Moreover, a polynucleotide or polypeptide that is introduced into an organism by transformation, genetic manipulation or by any other recombinant method is "isolated" even if it is still present in said organism, which organism may be living or non-living.

"Polynucleotide" generally refers to any polyribonucleotide (RNA) or polydeoxribonucleotide (DNA), which may be unmodified or modified RNA or DNA. "Polynucleotides" include, without limitation, single- and double-stranded DNA, DNA that is a mixture of single- and double-stranded regions, single- and double-stranded RNA, and RNA that is mixture of single- and double-stranded regions, hybrid molecules comprising DNA and RNA that may be single-stranded or, more typically, double-stranded or a mixture of single- and double-stranded regions. In addition, "polynucleotide" refers to triple-stranded regions comprising RNA or DNA or both RNA and DNA. The term "polynucleotide" also includes DNAs or RNAs containing one or more modified bases and DNAs or RNAs with backbones modified for stability or for other reasons. "Modified" bases include, for example, tritylated bases and unusual bases such as inosine. A variety of modifications may be made to DNA and RNA; thus, "polynucleotide" embraces chemically, enzymatically or metabolically modified forms of polynucleotides as typically found in nature, as well as the chemical forms of DNA and RNA characteristic of viruses and cells. "Polynucleotide" also embraces relatively short polynucleotides, often referred to as oligonucleotides.

"Polypeptide" refers to any polypeptide comprising two or more amino acids joined to each other by peptide bonds or modified peptide bonds, i.e., peptide isosteres. "Polypeptide" refers to both short chains, commonly referred to as peptides, oligopeptides or oligomers, and to longer chains, generally referred to as proteins. Polypeptides may contain amino acids other than the 20 gene-encoded amino acids. "Polypeptides" include amino acid sequences modified either by natural processes, such as post-translational processing, or by chemical modification techniques that are well known in the art. Such modifications are well described in basic texts and in more detailed monographs, as well as in a voluminous research literature. Modifications may occur anywhere in a polypeptide, including the peptide backbone, the amino acid side-chains and the amino or carboxyl termini. It will be appreciated that the same type of modification may be present to the same or varying degrees at several sites in a given polypeptide. Also, a given polypeptide may contain many types of modifications. Polypeptides may be branched as a result of ubiquitination, and they may be cyclic, with or without branching. Cyclic, branched and branched cyclic polypeptides may result from post-translation natural processes or may be made by synthetic methods. Modifications include acetylation, acylation, ADP-ribosylation, amidation, biotinylation, covalent attachment of flavin, covalent attachment of a heme moiety, covalent attachment of a nucleotide or nucleotide derivative, covalent attachment of a lipid or lipid derivative, covalent attachment of phosphotidylinositol, cross-linking, cyclization, disulfide bond formation, demethylation, formation of covalent cross-links, formation of cystine, formation of pyroglutamate, formylation, gamma-carboxylation, glycosylation, GPI anchor formation, hydroxylation, iodination, methylation, myristoylation, oxidation, proteolytic processing, phosphorylation, prenylation, racemization, selenoylation, sulfation, transfer-RNA mediated addition of amino acids to proteins such as arginylation, and ubiquitination (see, for instance, Proteins - Structure and Molecular Properties, 2nd Ed., T. E. Creighton, W. H. Freeman and Company, New York, 1993; Wold, F., Post-translational Protein Modifications: Perspectives and Prospects, 1-12, in Post-translational Covalent Modification of Proteins, B. C. Johnson, Ed., Academic Press, New York, 1983; Seifter *et al.,* "Analysis for protein modifications and nonprotein cofactors", Meth Enzymol, 182, 626-646, 1990, and Rattan *et al.,* "Protein Synthesis: Post-translational Modifications and Aging", Ann NY Acad Sci, 663, 48-62, 1992).

"Fragment" of a polypeptide sequence refers to a polypeptide sequence that is shorter than the reference sequence but that retains essentially the same biological function or activity as the reference polypeptide. "Fragment" of a polynucleotide sequence refers to a polynucloetide sequence that is shorter than the reference sequence of SEQ ID NO:1..

"Variant" refers to a polynucleotide or polypeptide that differs from a reference polynucleotide or polypeptide, but retains the essential properties thereof. A typical variant of a polynucleotide differs in nucleotide sequence from the reference polynucleotide. Changes in the nucleotide sequence of the variant may or may not alter the amino acid sequence of a polypeptide encoded by the reference polynucleotide. Nucleotide changes may result in amino acid substitutions, additions, deletions, fusions and truncations in the polypeptide encoded by the reference sequence, as discussed below. A typical variant of a polypeptide differs in amino acid sequence from the reference polypeptide. Generally, alterations are limited so that the sequences of the reference polypeptide and the variant are closely similar overall and, in many regions, identical. A variant and reference polypeptide may differ in amino acid sequence by one or more substitutions, insertions, deletions in any combination. A substituted or inserted amino acid residue may or may not be one encoded by the genetic code. Typical conservative substitutions include Gly, Ala; Val, Ile, Leu; Asp, Glu; Asn, Gln; Ser, Thr; Lys, Arg; and Phe and Tyr. A variant of a polynucleotide or polypeptide may be naturally occurring such as an allele, or it may be a variant that is not known to occur naturally. Non-naturally occurring variants of polynucleotides and polypeptides may be made by mutagenesis techniques or by direct synthesis. Also included as variants are polypeptides having one or more post-translational modifications, for instance glycosylation, phosphorylation, methylation, ADP ribosylation and the like. Embodiments include methylation of the N-terminal amino acid, phosphorylations of serines and threonines and modification of C-terminal glycines.

"Allele" refers to one of two or more alternative forms of a gene occuring at a given locus in the genome.

"Polymorphism" refers to a variation in nucleotide sequence (and encoded polypeptide sequence, if relevant) at a given position in the genome within a population.

"Single Nucleotide Polymorphism" (SNP) refers to the occurence of nucleotide variability at a single nucleotide position in the genome, within a population. An SNP may occur within a gene or within intergenic regions of the genome. SNPs can be assayed using Allele Specific Amplification (ASA). For the process at least 3 primers are required. A common primer is used in reverse complement to the polymorphism being assayed. This common primer can be between 50 and 1500 bps from the polymorphic base. The other two (or more) primers are identical to each other except that the final 3' base wobbles to match one of the two (or more) alleles that make up the polymorphism. Two (or more) PCR reactions are then conducted on sample DNA, each using the common primer and one of the Allele Specific Primers.

"Splice Variant" as used herein refers to cDNA molecules produced from RNA molecules initially transcribed from the same genomic DNA sequence but which have undergone alternative RNA splicing. Alternative RNA splicing occurs when a primary RNA transcript undergoes splicing, generally for the removal of introns, which results in the production of more than one mRNA molecule each of that may encode different amino acid sequences. The term splice variant also refers to the proteins encoded by the above cDNA molecules.

"Identity" reflects a relationship between two or more polypeptide sequences or two or more polynucleotide sequences, determined by comparing the sequences. In general, identity refers to an exact nucleotide to nucleotide or amino acid to amino acid correspondence of the two polynucleotide or two polypeptide sequences, respectively, over the length of the sequences being compared.

"% Identity" - For sequences where there is not an exact correspondence, a "% identity" may be determined. In general, the two sequences to be compared are aligned to give a maximum correlation between the sequences. This may include inserting "gaps" in either one or both sequences, to enhance the degree of alignment. A % identity may be determined over the whole length of each of the sequences being compared (so-called global alignment), that is particularly suitable for sequences of the same or very similar length, or over shorter, defined lengths (so-called local alignment), that is more suitable for sequences of unequal length.

"Similarity" is a further, more sophisticated measure of the relationship between two polypeptide sequences. In general, "similarity" means a comparison between the amino acids of two polypeptide chains, on a residue by residue basis, taking into account not only exact correspondences between a between pairs of residues, one from each of the sequences being compared (as for identity) but also, where there is not an exact correspondence, whether, on an evolutionary basis, one residue is a likely substitute for the other. This likelihood has an associated "score" from which the "% similarity" of the two sequences can then be determined.

Methods for comparing the identity and similarity of two or more sequences are well known in the art. Thus for instance, programs available in the Wisconsin Sequence Analysis Package, version 9.1 (Devereux J et al, Nucleic Acids Res, 12, 387-395, 1984, available from Genetics Computer Group, Madison, Wisconsin, USA), for example the programs BESTFIT and GAP, may be used to determine the % identity between two polynucleotides and the % identity and the % similarity between two polypeptide sequences. BESTFIT uses the "local homology" algorithm of Smith and Waterman (J Mol Biol, 147,195-197, 1981, Advances in Applied Mathematics, 2, 482-489, 1981) and finds the best single region of similarity between two sequences. BESTFIT is more suited to comparing two polynucleotide or two polypeptide sequences that are dissimilar in length, the program assuming that the shorter sequence represents a portion of the longer. In comparison, GAP aligns two sequences, finding a "maximum similarity", according to the algorithm of Needleman and Wunsch (J Mol Biol, 48, 443-453, 1970). GAP is more suited to comparing sequences that are approximately the same length and an alignment is expected over the entire length. Preferably, the parameters "Gap Weight" and "Length Weight" used in each program are 50 and 3, for polynucleotide sequences and 12 and 4 for polypeptide sequences, respectively. Preferably, % identities and similarities are determined when the two sequences being compared are optimally aligned.

Other programs for determining identity and/or similarity between sequences are also known in the art, for instance the BLAST family of programs (Altschul S F et al, J Mol Biol, 215, 403-410, 1990, Altschul S F et al, Nucleic Acids Res., 25:389-3402, 1997, available from the National Center for Biotechnology Information (NCBI), Bethesda, Maryland, USA and accessible through the home page of the NCBI at www.ncbi.nlm.nih.gov) and FASTA (Pearson W R, Methods in Enzymology, 183, 63-99, 1990; Pearson W R and Lipman D J, Proc Nat Acad Sci USA, 85, 2444-2448,1988, available as part of the Wisconsin Sequence Analysis Package).

Preferably, the BLOSUM62 amino acid substitution matrix (Henikoff S and Henikoff J G, Proc. Nat. Acad Sci. USA, 89, 10915-10919, 1992) is used in polypeptide sequence comparisons including where nucleotide sequences are first translated into amino acid sequences before comparison.

Preferably, the program BESTFIT is used to determine the % identity of a query polynucleotide or a polypeptide sequence with respect to a reference polynucleotide or a polypeptide sequence, the query and the reference sequence being optimally aligned and the parameters of the program set at the default value, as hereinbefore described.

"Identity Index" is a measure of sequence relatedness which may be used to compare a candidate sequence (polynucleotide or polypeptide) and a reference sequence. Thus, for instance, a candidate polynucleotide sequence having, for example, an Identity Index of 0.95 compared to a reference polynucleotide sequence is identical to the reference sequence except that the candidate polynucleotide sequence may include on average up to five differences per each 100 nucleotides of the reference sequence. Such differences are selected from the group consisting of at least one nucleotide deletion, substitution, including transition and transversion, or insertion. These differences may occur at the 5' or 3' terminal positions of the reference polynucleotide sequence or anywhere between these terminal positions, interspersed either individually among the nucleotides in the reference sequence or in one or more contiguous groups within the reference sequence. In other words, to obtain a polynucleotide sequence having an Identity Index of 0.95 compared to a reference polynucleotide sequence, an average of up to 5 in every 100 of the nucleotides of the in the reference sequence may be deleted, substituted or inserted, or any combination thereof, as hereinbefore described. The same applies *mutatis mutandis* for other values of the Identity Index, for instance 0.96, 0.97, 0.98 and 0.99.

Similarly, for a polypeptide, a candidate polypeptide sequence having, for example, an Identity Index of 0.95 compared to a reference polypeptide sequence is identical to the reference sequence except that the polypeptide sequence may include an average of up to five differences per each 100 amino acids of the reference sequence. Such differences are selected from the group consisting of at least one amino acid deletion, substitution, including conservative and non-conservative substitution, or insertion. These differences may occur at the amino- or carboxy-terminal positions of the reference polypeptide sequence or anywhere between these terminal positions, interspersed either individually among the amino acids in the reference sequence or in one or more contiguous groups within the reference sequence. In other words, to obtain a polypeptide sequence having an Identity Index of 0.95 compared to a reference polypeptide sequence, an average of up to 5 in every 100 of the amino acids in the reference sequence may be deleted, substituted or inserted, or any combination thereof, as hereinbefore described. The same applies *mutatis mutandis* for other values of the Identity Index, for instance 0.96, 0.97, 0.98 and 0.99.

The relationship between the number of nucleotide or amino acid differences and the Identity Index may be expressed in the following equation:

$$n_a \leq x_a - (x_a \bullet I),$$

in which:

$n_a$ is the number of nucleotide or amino acid differences,

$x_a$ is the total number of nucleotides or amino acids in SEQ ID NO:1 or SEQ ID NO:2, respectively,

I is the Identity Index ,

• is the symbol for the multiplication operator, and

in which any non-integer product of $x_a$ and I is rounded down to the nearest integer prior to subtracting it from $x_a$.

"Homolog" is a generic term used in the art to indicate a polynucleotide or polypeptide sequence possessing a high degree of sequence relatedness to a reference sequence. Such relatedness may be quantified by determining the degree of identity and/or similarity between the two sequences as hereinbefore defined. Falling within this generic term are the terms "ortholog", and "paralog". "Ortholog" refers to a polynucleotide or polypeptide that is the functional equivalent of the polynucleotide or polypeptide in another species. "Paralog" refers to a polynucleotideor polypeptide that within the same species which is functionally similar.

"Fusion protein" refers to a protein encoded by two, unrelated, fused genes or fragments thereof. Examples have been disclosed in US 5541087, 5726044. In the case of Fc- ANIC-BP-1 ligand, employing an immunoglobulin Fc region as a part of a fusion protein is advantageous for performing the functional expression of Fc- ANIC-BP-1 ligand or fragments of the ligand, to improve pharmacokinetic properties of such a fusion protein when used for therapy and to generate a dimeric ANIC-BP-1 ligand. The Fc- ANIC-BP-1 ligand DNA construct comprises in 5' to 3' direction, a secretion cassette, i.e. a signal sequence that triggers export from a mammalian cell, DNA encoding an immunoglobulin Fc region fragment, as a fusion partner, and a DNA encoding ANIC-BP-1 ligand or fragments thereof. In some uses it would be desirable to be able to alter the intrinsic functional properties (complement binding, Fc-Receptor binding) by mutating the functional Fc sides while leaving the rest of the fusion protein untouched or delete the Fc part completely after expression.

**Description of Figure**

**Figure 1:**

**Interaction of ANIC-BP-1 ligand with ANIC-BP-1 in S. cerevisiae EGY48/pSH18-32**

*S. cerevisiae* EGY48/18-32 (Clontech) was transformed using plasmids encoding LexA- and Gal4-activation domain fusion proteins as indicated in the cartoons. On the lower side of the figure. Single colonies were isolated and streaked onto Media lacking histidine, tryptophane and uracil with (-WHU-Xgal) or without (-WHU) 40 mg/l X-Gal and onto Medium lacking histidine, tryptophane, leucine and uracil containing 40 mg/l X-Gal(-WHLU-XGal).

Growth of all strains on -WHU-Medium indicates presence of plasmids. Blue colour of yeast and growth on Medium lacking leucine indicates interacion of baits and preys (Lane 5 and lane C) since expression of reportergenes depends on a Two-Hybrid selection scheme. Lanes 1-5 and lanes A-B are used as controlls. Lane C indicates interaction of ANIC and ANIC-BP1 in this Yeast Two-Hybrid System.

**Figure 2:**

S. cerevisiae Strain EGY48-pSH18-34 was transformed with plasmids encoding Fusion-proteins as indicated in the Figure. Activity of the β-Galactosidase-Reportergene was assayed using ONPG as described (Yeast Protocols Handbook, Clontech).

**Further examples.**

**Plasmid constructs:**

*Cloning of pDBLeu - ANIC-BP-1:*

The cDNA encoding ANIC-BP-1 was amplified by PCR using the primers ANICBP-Y2H2-up (SEQ ID NO: 3, primer 1) and ANICBP-Y2H-low (SEQ ID NO: 4, primer 2) and ligated into Vector pCR2.1TOPO (Invitrogen). Subsequently, the vector was cleaved using Nhe1 and Nco1 and the Mo25-encoding fragment was ligated into pDBLeu.

***Cloning* of *pLexA-MCS - ANIC-BP-1:***

Oligonucleotide-Primers Y2H-MCS1 (SEQ ID NO: 7, primer 3 ) and Y2H-MCS2 (SEQ ID NO: 8, primer 4) were annealed and ligated into EcoR1 and Sal1 restingated vector pLexA (Clontech) to generate Vector pLexA-MCS containing unique EcoR1, Sal1, Xho1 and Not1 restriction sites.

A fragment encoding ANIC-BP-1 was isolated from Vector pDBLeu-Mo25 using EcoR1 and Sal1 and ligated into Vector pLexA-MCS to generate pLexA-MCS-ANIC-BP-1. All vectors were confirmed by sequencing.

The peptide sequence of the Gal4-ANIC-BP-1 fusion protein comprising the Gal4 protein (position: 768-881) and a C-terminally linked full length ANIC-BP-1 protein sequence has been disclosed in Sequence ID No. 5. The corresponding peptide sequence of the LexA-ANIC-BP-1 fusion protein comprised the LexA protein sequence (position: 1-202) and a C-terminally linked full length ANIC-BP-1 protein sequence has been disclosed in Sequence ID No. 6.

**Yeast-Two Hybrid Screen to select ANIC-BP-1 interacting proteins.**

A yeast Two Hybrid screen (Proquest, Life Technologies) using pDBLeu-ANIC-BP-1 as bait construct was performed as described (selection was performed on -Trp, -Leu, -His Minimalmedium containing 25 mM 3-Aminotriazole). Interaction was confirmed by β-Galactosidase-filter assay and Uracile, Tryptophane and Leucine lacking medium as described in the manufacturers protocoll.

One positive clone was isolated and sequenced. The corresponding interacting protein ligand was termed ANIC-BP-1 ligand and the sequences have been disclosed in SEQ ID NO: 1 and NO: 2

Confirmation of the ANIC-BP-1/ANIC-BP-1 ligand interaction using a LexA-based Yeast Two-Hybrid selection scheme:

To confirm the interaction in a different selection scheme, the Matchmaker LexA Two-Hybrid system (Clontech) was used according to the manufacturers conditions. Vector pLexA-MCS-ANICBP was used as bait. Vector pPC86-ANICBPligand, which was isolated in the Gal4 based Yeast Two Hybrid system as described above was used as prey.

Interactions oft EphrinB2 with PICK1 and a novel PDZ-Domain containig protein were used as positve controlls in *S. cerevisiae* strain EGY48-pSH18-32. Colony growth on medium lacking Leucine and blue colony colour indicates interaction of bait and prey proteins.

## Claims

1. An isolated ANICP-BP-1 ligand protein, which binds to a fusion protein comprising the ANIC-BP-1 sequence of SEQ IDs 5 and/or 6 selected from the group consisting of:

   (a) a polypeptide encoded by a polynucleotide comprising the sequence of SEQ ID NO:1;
   (b) a polypeptide comprising a polypeptide sequence having at least 95% identity to the polypeptide sequence of SEQ ID NO:2;
   c) a polypeptide having at least 95% identity to the polypeptide sequence of SEQ ID NO:2;
   d) the polypeptide sequence of SEQ ID NO:2.

2. An isolated polynucleotide selected from one of the groups consisting of:

   (a) an isolated polynucleotide comprising a polynucleotide sequence having at least 95% identity to the polynucleotide sequence of SEQ ID NO:1;
   (b) an isolated polynucleotide having at least 95% identity to the polynucleotide of SEQ ID NO:1;
   (c) an isolated polynucleotide comprising a polynucleotide sequence encoding a polypeptide sequence having at least 95% identity to the polypeptide sequence of SEQ ID NO:2;
   (d) an isolated polynucleotide having a polynucleotide sequence encoding a polypeptide sequence having at least 95% identity to the polypeptide sequence of SEQ ID NO:2;
   (e) an isolated polynucleotide which is the RNA equivalent of a polynucleotide of (a) to (d);

   or a polynucleotide sequence complementary to said isolated polynucleotide over the entire length thereof.

3. An isolated expression system comprising a polynucleotide capable of producing a polypeptide of any one of claim 1-2 when said expression vector is present in a compatible host cell.

4. A non-human recombinant host cell comprising the expression vector of claim 3 or a membrane thereof expressing the polypeptide of any one of claim 1-2

## Patentansprüche

1. **1.** Isoliertes ANICP-BP-1-Ligandenprotein, das an ein Fusionsprotein bindet, enthaltend die ANIC-BP-1-Sequenz von SEQ ID 5 und/oder 6, ausgewählt aus der Gruppe, bestehend aus:

   (a) einem Polypeptid, das von einem Polynukleotid kodiert wird, das die Sequenz von SEQ ID NO: 1 enthält;
   (b) einem Polypeptid, enthaltend eine Polypeptidsequenz mit mindestens 95% Identität zur Polypeptidsequenz von SEQ ID NO:2;
   (c) einem Polypeptid mit mindestens 95% Identität zur Polypeptidsequenz von SEQ ID NO:2;
   (d) der Polypeptidsequenz von SEQ ID NO:2.

2. **2.** Isoliertes Polynukleotid, ausgewählt aus einer der Gruppen, bestehend aus:

   (a) einem isolierten Polynukleotid, enthaltend eine Polynukleotidsequenz mit mindestens 95% Identität zur Polynukleotidsequenz von SEQ ID NO:1;
   (b) einem isolierten Polynukleotid mit mindestens 95% Identität zum Polynukleotid von SEQ ID NO:1;
   (c) einem isolierten Polynukleotid, enthaltend eine Polynukleotidsequenz, die eine Polypeptidsequenz mit mindestens 95% Identität zur Polypeptidsequenz von SEQ ID NO:2 kodiert;
   (d) einem isolierten Polynukleotid mit einer Polynukleotidsequenz, die eine Polypeptidsequenz mit mindestens 95% Identität zur Polypeptidsequenz von SEQ ID NO:2 kodiert;
   (e) einem isolierten Polynukleotid, das das RNA-Äquivalent eines Polynukleotids von (a) bis (d) ist.

   oder eine Polynukleotidsequenz, die über ihre gesamte Länge zum isolierten Polynukleotid komplementär ist.

3. **3.** Isoliertes Expressionssystem, enthaltend ein Polynukleotid, das ein Polypeptid nach einem der Ansprüche 1 bis 2 erzeugen kann, wenn der Expressionsvektor in einer kompatiblen Wirtszelle zugegen ist.

4. **4.** Nicht-humane rekombinante Wirtszelle, enthaltend den Expressionsvektor nach Anspruch 3, oder eine Membran davon, welche das Polypeptid nach einem der Ansprüche 1 bis 2 exprimiert.

## Revendications

1. **1.** Protéine de ligand ANICP-BP-1 isolée, laquelle se lie à une protéine de fusion comprenant la séquence ANIC-BP-1 des SEQ IDs 5 et/ou 6 choisie parmi la groupe comprenant :

   (a) un polypeptide codé par un polynucléotide comprenant la séquence de SEQ ID NO:1 ;
   (b) un polypeptide comprenant une séquence polypeptidique ayant une identité d'au moins 95% à la séquence polypeptidique de SEQ ID NO:2;
   (c un polypeptide ayant une identité d'au moins 95% à la séquence polypeptidique de SEQ ID NO:2 ;
   (d) la séquence polypeptidique de SEQ ID NO:2.

2. **2.** Polynucléotide isolé choisi parmi un élément du groupe constitué par :

   (a) un polynucléotide isolé comprenant une séquence polynucléotidique ayant une identité d'au moins 95% à la séquence polypeptidique de SEQ ID NO:1 ;
   (b) un polynucléotide isolé ayant une identité d'au moins 95% au polynucléotide de SEQ ID NO:1 ;
   (c) un polynucléotide isolé comprenant une séquence polynucléotidique codant une séquence polypeptidique ayant une identité d'au moins 95% à la séquence polypeptidique de SEQ ID NO:2 ;
   (d) un polynucléotide isolé ayant une séquence polynucléotidique codant une séquence polypeptidique ayant une identité d'au moins 95% à la séquence polypeptidique de SEQ ID NO:2 ;
   (e) un polynucléotide isolé qui est l'équivalent ARN d'un polynucléotide de (a) à (d);

   ou une séquence polynucléotidique complémentaire dudit polynucléotide isolé ou la longueur entière afférente.

3. **3.** Système d'expression isolée comprenant un polynucléotide capable de produire un polypeptide selon l'une quelconque des revendications 1-2 lorsque ledit vecteur d'expression est présent dans une cellule hôte compatible.

**4.** Cellule hôte recombinante non humaine comprenant le vecteur d'expression selon la revendication 3 ou une membrane afférente exprimant le polypeptide selon l'une quelconque des revendications 1-2.

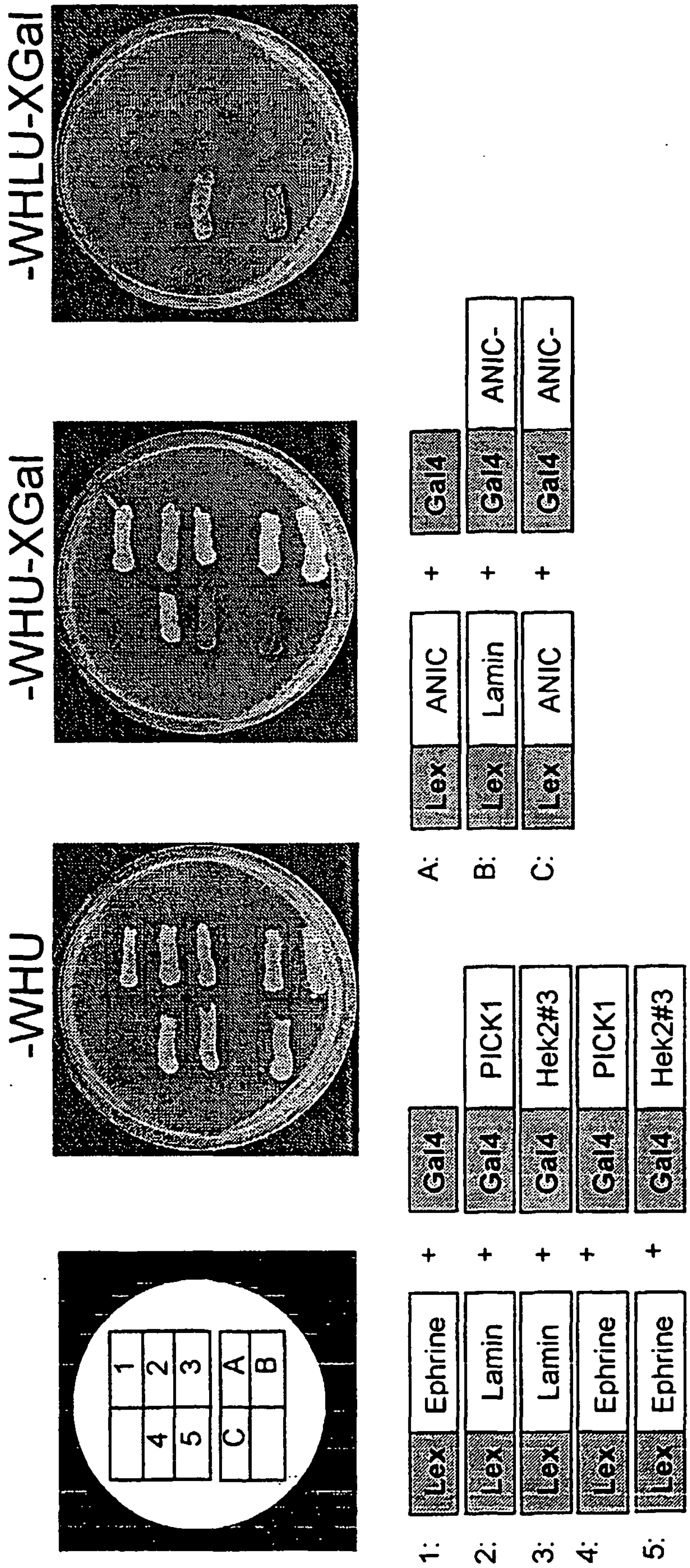


Figure 1 from 2

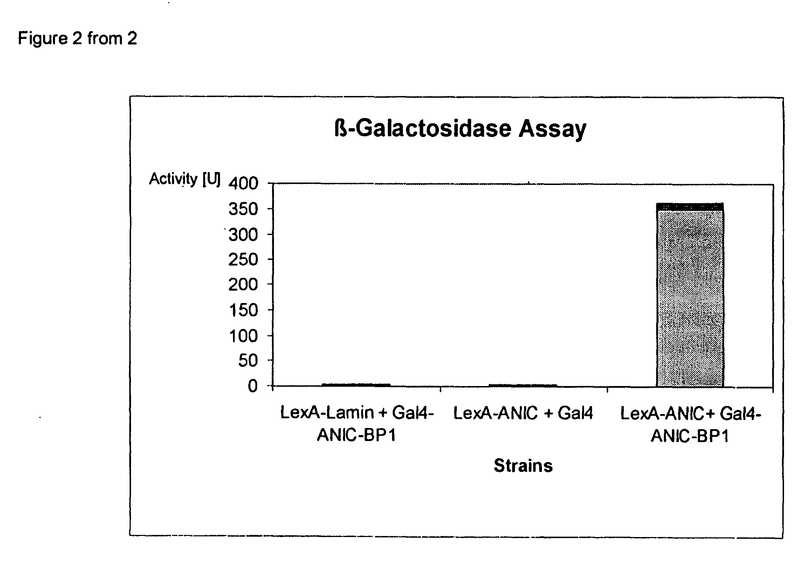

Figure 2 from 2